# EUROPEAN PATENT APPLICATION

(11) **EP 1 762 179 A1**
(43) Date of publication of application: **14.03.2007**
(21) Application number: 05108190.9
(22) Date of filing: 07.09.2005
(51) Int. Cl.: A61B 8/06

(54) **Method for improving diagnostic quality in echocardiography**

(71) Applicant: BOEHRINGER INGELHEIM VETMEDICA GMBH, 55216 Ingelheim am Rhein (DE)
(72) Inventor: Stark, Marcus, 65203, Weisbaden (DE); Kleemann, Rainer, 55218, Ingelheim (DE); Dämmgen, Jürgen, 88416, Ochsenhausen (DE)
(74) Representative: Hammann, Heinz

(57) **Abstract**

The present invention provides a method for improving diagnostic quality in echocardiography, especially for patients having an elevated heart rate.

## Description

### FIELD OF THE INVENTION

The present invention relates to a method for improving diagnostic quality in echocardiography, especially for patients having an elevated heart rate.

### BACKGROUND OF THE INVENTION

Echography of the heart (echocardiography - ECG) has become a major diagnostic tool as non invasive method in human as well as veterinary medicine. The techniques used are two-dimensional-, motion-mode- and Doppler-ECG. An overview of these techniques can be found in Moise N.S. and Fox P.R., Echocardiography and Doppler Imaging (Fox P.R., Sisson D., Moise N.S. ed., Textbook of Canine and Feline Cardiology, 2nd edition 1999, pp. 130 and ff, W.B. Saunders Company, Philadelphia, USA).

Many parameters measured with ECG are potentially influenced by high heart rates. High heart rates can diminish ECG quality and even make important measurements impossible.

The most common heart disease in cats is hypertrophic cardiomyopathy (HCM). Abnormal left ventricular (LV) relaxation is a common manifestation of HCM. A non-invasive and practical technique to assess LV relaxation is to measure transmitral (blood)flow characteristics through Doppler ECG. The most characteristic Doppler echo feature of LV relaxation abnormality is a low E velocity (**e**arly, passive diastolic filling) with prolonged deceleration, and high A velocity (**a**trial contraction). Unfortunately, in cats the E and A waves are commonly not separated because of their relatively fast heart rates, "making this technique useless", or at least "interpretation difficult". This is reflected in the articles of Kienle R.D., Echocardiography (Kittleson M.D., Kienle R.D. ed., Small Animal Cardiovascular Medicine, 1998, pp. 95 and ff, Mosby, Inc., St. Louis, USA) and Fox P.R., Feline Cardiomyopathies (Fox P.R., Sisson D., Moise N.S. ed., Textbook of Canine and Feline Cardiology, 2nd edition 1999, pp.621 and ff., W.B. Saunders Company, Philadelphia, USA).

However, the measurement of E and A wave and calculated E/A ratios are very useful parameters for diagnosis, prognosis and treatment of diastolic dysfunction in human patients, and potentially in cats.

Elevated heart rate, especially in pet animals, may be treated with bradycardic agents such as calcium (Ca⁺⁺) channel blockers, beta-receptor blockers and (**l_{f}**) channel blockers.

Known Ca⁺⁺ channel blockers which may be used for this purpose are diltiazem and verapamil.

Known beta-receptor blockers which may be used for this purpose are atenolol, bisoprolol, carvedilol, metoprolol or propanolol.

Known l_{f} channel blockers which may be used for this purpose are zatebradine [1-(7,8-dimethoxy-1,3,4,5-tetrahydro-2H-3-benzazepin-2-on-3-yl)-3-[N-methyl-N-(2-(3,4-dimethoxy-phenyl)-ethyl)-propane], disclosed for example in EP-B-0 065 229 and its US counterpart US 5,516,773, cilobradine (3-[(N-(2-(3,4-dimethoxy-phenyl)-ethyl)-piperidin-3-yl)-methyl]-(7,8-dimethoxy-1,3,4,5-tetrahydro-2H-3-benzazepin-2-one) and its hydrochloride salt, disclosed for example in EP-B-0 224 794 and its US counterpart US 5,175,157, alinidine [2-(N-allyl-2,6-dichloro-anilino)-2-imidazolidine), disclosed for example in US 3,708,485, or ivabradine 3-[3-[[[(7S)-3,4-dimethoxybicyclo[4.2.0]octa-1,3,5-trien-7-yl]methyl]methylamino]propyl]-1,3,4,5-tetrahydro-7,8-dimethoxy-2H-3-benzazepin-2-one and its hydrochloride salt, disclosed for example in EP-B-534859.

Zatebradine is also known to have a favourable activity in the treatment of cardiac insufficiency (see EP-B-0 471 388). Cilobradine is also known to have a favourable activity in the treatment or prevention of heart failure (see EP-B-1 362 590). Cilobradine, zatebradine and alinidine are also known to have a favourable activity in the treatment and induction of the regression of idiopathic hypertrophic cardiomyopathy (HCM), ischemic cardiomyopathy and valvular hypertrophic heart diseases (see WO 01/78699). Ivabradine is especially known to have a favourable activity in the treatment of myocardial disorders (from EP-B-534859 or US 5,296,482).

Scientific studies performed with zatebradine and cilobradine in order to determine the mechanism of action of these bradycardic substances have shown that both zatebradine and cilobradine selectively block the hyperpolarisation activated, cAMP-modulated cation current channels (HCN) in cardiac conductive tissues, the channels responsible for the transmembrane current known as l. It is through blockade of this current that zatebradine and cilobradine are assumed to produce their specific bradycardic effect.

### SUMMARY OF THE INVENTION - PREFERRED EMBODIMENTS

The present invention relates to a method for improving diagnostic quality in echocardiography, especially for patients having an elevated heart rate. This invention thus relates both to diagnostic and to the retrieval of quality improved information for diagnostic purposes.

Surprisingly, it has been found that the administration of a substance which has the ability to reduce heart rate (bradycardic substance) to a patient subject to ECG allows to reliably measure certain ECG parameters (e.g. transmitral flow characteristics through Doppler) which were impossible or difficult to assess before (i.e. without the administration of the bradycardic substance).

The present invention thus relates to a method for improving diagnostic quality in echocardiography, said method comprising the administration of a bradycardic substance to the patient subject to the echocardiography.

The present invention further relates to a method for retrieving quality improved information in echocardiography, said method comprising the administration of a bradycardic substance to the patient subject to the echocardiography.

The present invention further relates to a quality improvement in a method for performing an echocardiography, said improvement comprising the administration of a bradycardic substance to the patient subject to the echocardiography.

The present invention further relates to the use of a bradycardic substance for the preparation of a composition for improving the diagnostic quality in echocardiography.

The present invention further relates to a composition for improving the diagnostic quality in echocardiography, said composition comprising a bradycardic substance.

Within the meaning of the present invention, the above composition, method or use is intended for humans as well as non-human patients. In a further embodiment, the above composition, method or use is intended for non-human patients, i.e. for the veterinary field, preferably for mammals, more preferably for pet animals, even more preferably for cats.

In a further embodiment, the above composition, method or use is intended for improving the quality in echocardiography for patients having an elevated heart rate.

In a further embodiment, the bradycardic agent is selected from calcium (Ca⁺⁺) channel blockers, beta-receptor blockers and (**l**_{f}) channel blockers.

In a further embodiment, the bradycardic agent is selected from the following calcium (Ca⁺⁺) channel blockers: diltiazem and verapamil.

In a further embodiment, the bradycardic agent is selected from the following beta-receptor blockers: atenolol, bisoprolol, carvedilol, metoprolol or propanolol.

In a further embodiment, the bradycardic agent is selected from the following l_{f} channel blockers: zatebradine, cilobradine and its hydrochloride salt, alinidine, and ivabradine and its hydrochloride salt.

Within the meaning of the present invention, the bradycardic agent can be used in the form of one of its salts, preferably one of its pharmaceutically acceptable salts. Suitable known pharmaceutically acceptable salts of the above-mentioned bradycardic agents are, for example, the hydrochloride salt of cilobradine or ivabradine.

Amongst the bradycardic agents to be used within the meaning of the present invention, the l_{f} channel blocker cilobradine, and especially its hydrochloride salt, is preferred.

### DETAILLED DESCRIPTION OF THE INVENTION

Suitable bradycardic agents for the practice of the invention have been disclosed hereinbefore. Pharmaceutically acceptable salts or esters of these agents may also be employed. The pharmaceutically acceptable salts of some bradycardic agents suitable for the practice of the invention have been disclosed hereinbefore.

The preparation of the bradycardic agents or their pharmaceutically acceptable salts or esters suitable for the practice of the invention is known per se. For example, for the preparation of cilobradine or its pharmaceutically acceptable salts, reference is made to the aforementioned literature EP-B-0 224 794 and its US counterpart US 5,175,157, which describes the chemical synthesis of these compounds. For the preparation of zatebradine or its pharmaceutically acceptable salts, reference is made to the aforementioned literature EP-B-0 065 229 and its US counterpart US 5,516,773, which describes the chemical synthesis of these compounds. For the preparation of ivabradine or its pharmaceutically acceptable salts, reference is made to the aforementioned literature EP-B-0 534 859, which describes the chemical synthesis of these compounds.

For the practice of the invention, the bradycardic agent may be administered to patients in any medically or veterinary acceptable manner. Hence, the composition comprising the bradycardic agent may be formulated as liquid formulation or lyophilised powder for oral or parenteral administration. Powders may be reconstituted by addition of a suitable diluent or other pharmaceutically acceptable carrier prior to use. The liquid formulation is generally an aqueous solution. Such formulation is especially suitable for oral administration, but may also be used for parenteral administration or contained in a metered dose inhaler or nebulizer for insufflation. It may be desirable to add excipients such as polyvinylpyrrolidone or hydroxycellulose to the composition. The liquid formulation may be administered directly per orally or filled into a soft capsule. Alternatively, the ingredients may be encapsulated, tableted or prepared in a syrup for oral administration. Pharmaceutically acceptable solid or liquid carriers may be added to enhance or stabilise the composition, or to facilitate the preparation of the composition. The carrier may also include a sustained release material. The compositions are prepared following the conventional techniques of pharmacy involving milling, mixing, granulating, and compressing, when necessary, for tablet forms, or milling, mixing and filling for capsule forms.

For the purposes of practicing the current invention it is however preferred that the bradycardic agent be administered orally or via injection, the oral route being preferred. Suitable compositions for the oral administration of the aforementioned agents are known per se. For example, a suitable composition for the administration of alinidine and ivabradine is disclosed in WO 02/45693, to which reference is made. For the preparation of compositions comprising cilobradine or its pharmaceutically acceptable salts, reference is made in particular to EP-B-0 224 794 and its US counterpart US 5,175,157, to WO 01/78699 and to EP-B-1 362 590, which describe examples of injectable, oral liquid, tablet, capsule and suppository formulations of cilobradine or its pharmaceutically acceptable salts.

In order to achieve the effect according to the invention it is expedient to use the dosages known from the literature for the treatment of elevated heart rate for the individual bradycardiac agents, dosages which are known to those of ordinary skill in the medical or veterinary art and to which reference is made, or the dosages evaluated in appropriate studies for each of these bradycardic agents.

For example, the following single dosages are known from the literature:
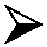 for cilobradine, the single dose is 0.1 to 0.5 mg/kg per os, preferably 0.2 to 0.4 mg/kg, 1 to 3 x daily;
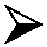 for zatebradine, the single dose is 0.2 to 1 mg/kg 2 x daily; and
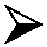 for alinidine, the single dose is 0.5 to 5 mg/kg 2 x daily.

For example, the following dosages relevant for practicing the present invention with the bradycardic agent cilobradine in cats and dogs, have been determined in appropriate pharmacokinetic studies performed by the inventors. The single oral dose of cilobradine for potent heart rate reduction in cats is 0.2 - 0.4 mg/kg. The single oral dose of cilobradine for potent heart rate reduction in dogs is 0.3 mg/kg. Absolute bioavailabilities of oral formulations of cilobradine are 22.6% in cats and 26 - 43% in dogs. Peak plasma levels are reached 0.5 hours after oral administration in cats and after 1 to 2 hours in dogs.

Within the meaning of the present invention, the following dosage ranges are preferred for the bradycardic agent cilobradine: 0.05 to 5 mg/kg body weight, 0.1 to 2.5 mg/kg body weight, 0.1 to 1 mg/kg body weight, and 0.1 to 0.75 mg/kg body weight.

The invention will now be described in more detail with reference to the following experiment.

The use according to the invention of an l_{f} channel blocker, namely cilobradine, was investigated in a clinical field study with cats suffering from asymptomatic HCM.

Four cats diagnosed as suffering from asymptomatic HCM by means of motion mode ECG (left ventricular wall thickness > 6mm), received cilobradine orally once daily for 5 consecutive days. Detailed clinical examinations were conducted at baseline and on days 1, 2, 3, 6 and 9. ECG measurements were performed at baseline and on days 3 and 6 of the study.

The composition used in the experiment was as follows.
Oral solution containing 2 mg/ml cilobradine
Active ingredient added to 0.9% NaCl solution

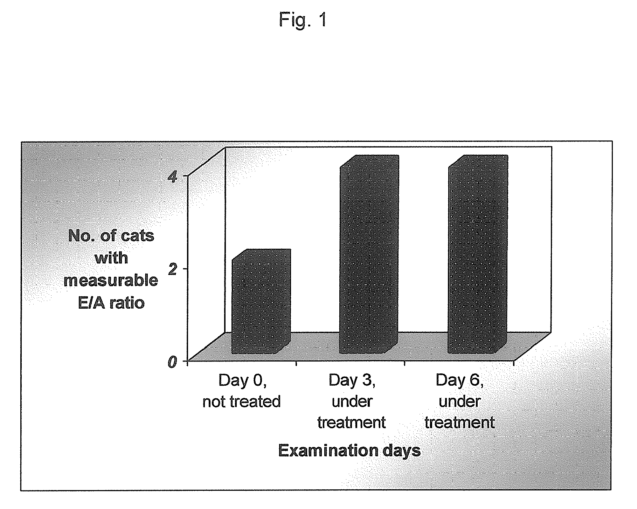

The results of the experiment, measuring the left ventricular E to A ratios obtained at baseline and under cilobradine treatment, are shown in enclosed Figure 1. These results show that calculation of left ventricular E/A ratios was not possible in 2 of 4 cats at baseline, thus before treatment with cilobradine. During treatment with cilobradine, at days 3 and 6, calculation of left ventricular E/A ratios was possible in all cats.

From this study, it can be concluded that treatment with the l channel blocker cilobradine facilitates and improves the quality of ECG diagnosis.

## Claims

1. A method for improving diagnostic quality in echocardiography, said method comprising the administration of a bradycardic substance to a human or non-human patient subject to an echocardiography.

2. The method in accordance with claim 1, wherein the patient has an elevated heart rate.

3. The method in accordance with claim 1 or 2, wherein the patient is a pet animal.

4. The method in accordance with any one of claims 1 to 3, wherein the bradycardic agent is an l_{f} channel blocker.

5. The method in accordance with any one of claims 1 to 4, wherein the bradycardic agent is the l_{f} channel blocker cilobradine or a pharmaceutically acceptable salt thereof.

6. Use of a bradycardic substance for the preparation of a composition for improving the diagnostic quality of a human or non-human patient subject to an echocardiography.

7. Use in accordance with claim 6, wherein the patient has an elevated heart rate.

8. Use in accordance with claim 6 or 7, wherein the patient is a pet animal.

9. Use in accordance with any one of claims 6 to 8, wherein the bradycardic agent is an l_{f} channel blocker.

10. Use in accordance with any one of daim 6 to 9, wherein the bradycardic agent is the channel blocker cilobradine or a pharmaceutically acceptable salt thereof.
